# EUROPEAN PATENT APPLICATION

(11) **EP 0 531 597 A1**
(43) Date of publication of application: **17.03.1993**
(21) Application number: 91402427.8
(22) Date of filing: 12.09.1991
(51) Int. Cl.: C07F 9/6561, A61K 31/675, C07F 9/6512

(54) **Novel unsaturated acyclic phosphonate derivatives of purine and pyrimidine**

(71) Applicant: MERRELL DOW PHARMACEUTICALS INC., Cincinnati Ohio 45215-6300 (US)
(72) Inventor: Halazy, Serge, F-67200 Strasbourg (FR); Danzin, Charles, F-67000 Strasbourg (FR); Nave, Jean-François, F-67000 Strasbourg (FR)
(74) Representative: Gillard, Marie-Louise

(57) **Abstract**

This invention relates to novel unsaturated acyclic phosphonate derivatives of certain purine or pyrimidines useful as anti-viral agents and as purine nucleoside phosphorylase inhibitors, to methods and intermediates useful for their preparation and to their end-use application as immunosuppressants, anti-lymphoma, anti-leukemic, anti-viral and antiprotozoal agents and also as agents used in conjunctive therapy to potentiate the efficacy of anti-viral nucleoside analogs which would otherwise become subject to the enzymative action of purine nucleoside phosphorylase.

## Description

This invention relates to novel unsaturated acyclic phosphonate derivatives of certain purine or pyrimidines useful as anti-viral agents and as purine nucleoside phosphorylase inhibitors, to methods and intermediates useful for their preparation and to their end-use application as immunosuppressants, anti-lymphoma, anti-leukemic, anti-viral and antiprotozoal agents and also as agents used in conjunctive therapy to potentiate the efficacy of anti-viral nucleoside analogs which would otherwise become subject to the enzymative action of purine nucleoside phosphorylase.

More specifically this invention relates to novel compounds of the formula 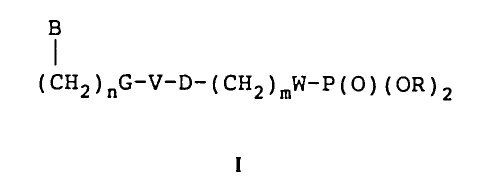
the isomeric and tautomeric forms thereof, and the pharmaceutically acceptable salts thereof, wherein B is a purine or pyrimidine moiety of the formulae 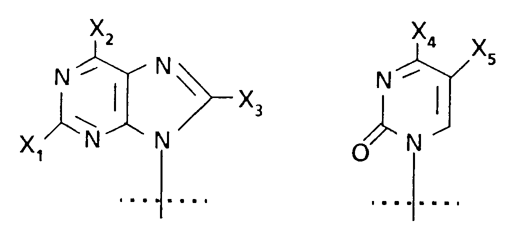
wherein
- X₁: is H, NH₂, F, Cl, Br or I,
- X₂: is H, OH, Cl, NH₂, SCH₃, SH, NHCH₃, or NHC₃₋₆ cycloalkyl,
- X₃: is H, OH, NH₂, NHNH₂, CH₃, Cl, Br, or NHCH₃,
- X₄: is NH₂, OH, OC₁₋₃ alkyl,
- X₅: is H, CH₃, C₂H₅, I, F, Cl, Br, NHR, SR, C≡CH, CH=CH₂, CH=CHBr, CH₂CH₂Cl, or CH₂CH₂F,
- R: is H or C₁₋₈ alkyl,
- m: and n are zero, 1, 2 3 or 4,
- V: is 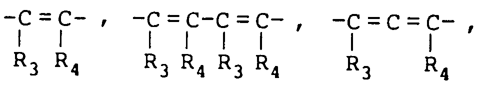 -C≡C- or 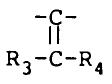
- R₃: is H, F, Cl, CH₃, CH₂OH, CH₂F or OH,
- R₄: is H, F, Cl, CH₃, CH₂OH, CH₂F or OH,
- G: is 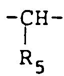 or is deleted,
- D: is 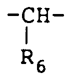 or is deleted with the proviso that at least one of G and D is deleted,
- R₅: is F, Cl, CH₃, CH₂OH, OH or CH₂F with the proviso that when n is zero R₅ is other than OH, F or Cl, and
- R₆: is F, Cl, CH₃, CH₂OH, OH or CH₂F,
- W: is OCH₂, CF₂, CCl₂, CHF, CHCl or is deleted.

As used herein, the term alkyl includes the straight and branched chain saturated hydrocarbyl radicals (having the indicated number of carbon atoms) which include methyl, ethyl, propyl, isopropyl, butyl, t-butyl, pentyl, hexyl and the like. C₃₋₆ cycloalkyl radicals include such as cyclopropyl, cyclopentyl, cyclobutyl and cyclohexyl.

For clarity, the dotted line beneath the purine and pyrimidine bases indicates the bond through which the remaining portion of the molecule is attached to the N-9-position of the purine moiety or to the N-1-position of the pyrimidine moiety.

The term "pharmaceutically acceptable salts" embraces both acid addition salts and metal and amine salts which are known to be non-toxic and useful derivatives in the preparation of pharmaceutical formulations suitable for end-use applications.

Pharmaceutically acceptable acid addition salts include the known non-toxic organic or inorganic acid addition salts of the base compounds of Formula **I**. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic forms, demonstrate higher melting points and an increased stability.

Pharmaceutically acceptable metal and amine salts are those salts which are stable under ambient conditions, and wherein the cation does not contribute significantly to the biological activity of the salt. Suitable metal salts include the sodium, potassium, calcium, barium, zinc, and aluminium salts. The sodium and potassium salts are preferred. Suitable amine salts are prepared from amines which have sufficient basicity to form a stable salt, and preferably include those amines which are frequently used in medicinal chemistry because of their low toxicity and acceptability for medical use. These include the trialkylamines such as triethylamine, and others including procaine, dibenzylamine. N-benzyl-betaphenethylamine, ephenamine, and N,N'-dibenzylethylenediamine, dehydroabietylamine, N-ethylpiperidine, benzylamine, and dicyclohexylamine.

The isomeric forms of the compounds of Formula **I** include the optical and geometric forms which are well-known entities and may be prepared by known processes designed to enrich the production of the desired geometrical isomers and/or optical isomers. However, mixtures may be resolved or isolated according to conventional and standard procedures well known in the art, e.g., chromatographic separation, fractional crystallization, use of optically active acids, enzymatic resolution and the like. Tautomeric enol-keto forms may exist at the 6-position of the purine nucleus, and the pyrimidine will exhibit amine-imine tautomeric forms.

For convenience, the moieties of V, i.e. 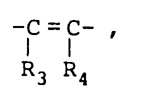 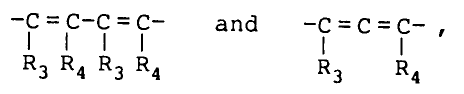
may sometimes be written as -C(R₃)=C(R₄), -C(R₃)=C(R₄)-C(R₃)=C(R₄), and -C(R₃)=C=C(R₄), respectively.

It should also be obvious that the reading of the individual components of the -(CH₂)ₙG-V-D-(CH₂)ₘW- moiety should be as they are depicted in their relative positions to the B and the P(O)(OR)₂ moieties. For example, if W is OCH₂ it is correct to interpret its relative positioning as -OCH₂P(O)(OR)₂ whereas it would be incorrect to interpret the resulting moiety as CH₂-O-P(O)(OR)₂. Similar circumstances prevail with the V moieties.

Preferred pyrimidine type bases are uracil, thymine, cytidine, 5-halogenated (F, Br or I) or 5-carboxy uracils, and 5-methyl or 5-hydroxymethylcytidines. Preferred purine type bases are adenine, guanine, 6-methylamino or 6,6-dimethylamino purines or 6-OH purines, 8-azaguanine, and 6-oxy or 2,6-dioxy purines. Otherwise stated, preferred compounds of this invention are those which contain such nucleosidic moieties as adenosine, deoxyadenosine, guanosine, deoxyguanosine, cytidine, deoxycytidine, uridine, deoxyuridine, thymidine, inosine and xanthosine.

The compounds of this invention may be prepared by the application of analogous chemical reactions known in the art, using reactants which are already known or which may be prepared using standard processes and techniques known in the art. In its essence, the general method for preparing the compounds of Formula **I** may be depicted by the following reaction scheme. 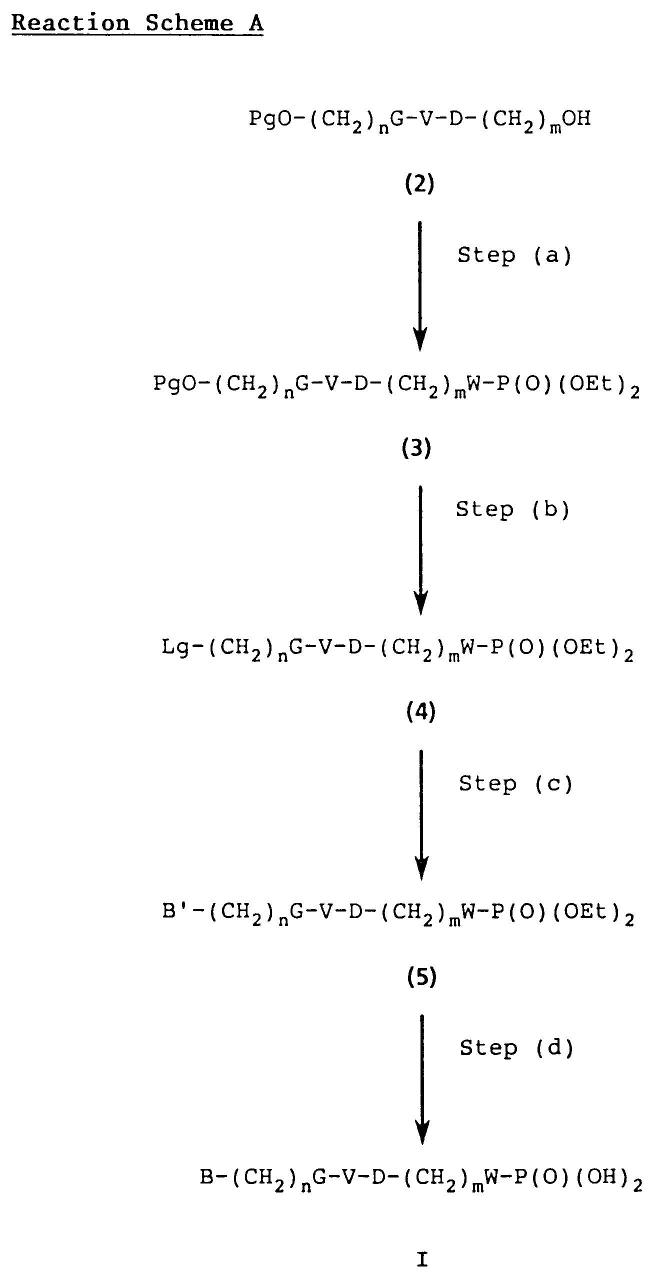
wherein Pg is an alcohol protecting group, Lg is a suitable leaving group, B' is B or a moiety transformable thereto, B, G, V and D are as defined, Et is ethyl but other radicals (e.g. C₁₋₈ alkyls, particularly isopropyl, phenyl, benzyl or phenylalkyl) known to be useful to protect the phosphonic acid moiety may also be used. Of course, any of the G, V and D moieties may also be selectively protected for any particular reaction wherein that moiety would interfere with the efficiency of the desired condensation or other reaction.

The foregoing reaction scheme illustrates a series of standard and well-known reaction steps which are generally applicable to those compounds of Formula **I** wherein the purine or pyrimidine nucleic bases are not attached directly to a carbon atom bearing an unsaturation. Suitable leaving groups (Lg) for these reactions include such groups as triflates, alkyl or aryl sulfonates (e.g. CF₃SO₂O, tosylate, mesylate) and halides (Br, Cl, or I). Suitable metallic cations which are utilized in effecting the required condensations are Na, K, Li, Cs, Mg, Mg Br or Zn Br. Suitable protecting groups include the known esters and ethers, with acetyl, benzoyl, benzyl and tetrahydropyranyl being prepared. Of course, when necessary, selective hydroxy protecting groups, e.g. t-butyl dimethyl silyl ether, may be used when their removal is necessary prior to removal of the depicted protecting groups of the terminal hydroxy function of Formulae **(2)** and **(3)**. The B' moieties represent either the X₁, X₂, X₃- substituted purines or the X₄, X₅-substituted pyrimidines of Formula **I** or they are X'₁, X'₂, X'₃- substituted purines or the X'₄, X'₅- substituted pyrimidines which are transformable thereto by standard techniques using well-known processes. For example, if an X moiety is to chloro in the final product then the X' moiety would be chloro for that would be left intact throughout the entire series of reactions for any given compound. On the other hand, one could utilize a 6-chloro or a 2,6-dichloro purine starting compound and after the series of reactions is completed they could be transformed into a 6-amino or a 2,6-diamino final product, respectively by reaction with the appropriate amines [i.e.,NH₃, -HN(H)(R₂) or -HN(R₂)(R₂)] in methanol under pressure at about 100°C. Similarly, in those instances wherein X is OR, the corresponding 6-chloro purine moiety may be reacted with an acid (HCl/HOH) to obtain the corresponding 6-OH analog or with an alcohol in basic conditions to obtain the corresponding 6-OR analog. In the same vein, X' may be a protected OH group using such groups as -OCH₃, OCH₂CH₂OCH₃, Obenzyl, O-C(O)phenyl, or -O-C(O)methyl and then such moieties would be transformed to the desired 6-OH moieties. Analogously, a compound wherein an X- substituent is to be an amine, the precursor transformable theretocould be an amide (e.g., NHC(O)phenyl, an amidine (e.g.-N=CHN〈 ) or -N(C(O) phenyl)₂, said moieties being transformable to the desired amine by standard procedures. Similarly, a 6-chloro purine starting material may be transformed to the desired -SCH₃ moiety by treatment with methyl mercaptan using standard procedures. These reactions, and the proper sequencing of such reactions, all follow principles well understood and practiced by those skilled in the art and all steps used processes well known in the art, as well as other factors such as the ready availability of the appropriate starting materials, reactants and the volume capacity of compound to be produced are also factors which are appreciated by those skilled in the art for choosing any particular reaction scheme.

Typically in the preparation of the compounds produced by Reaction scheme A, the key process steps entail condensation reactions of an activated nucleophile with an electrophile bearing a suitable leaving group, such condensations being utilized in Steps (a) and (c). Step (b) represents a deprotection and replacement of the protecting group with a suitable leaving group. Step (d) involves the de-esterification of the phosphonate moiety and the transformation, if needed, of the X'₁, X'₂, X'₃, X'₄ and X'₅ moieties to their respective X₁, X₂, X₃, X₄ and X₅ moieties. Optionally, the de-esterification may be effected after the transformation of the X moieties.

In effecting the condensation the reactants are contacted together in a suitable anhydrous solvent (e.g. THF, DMF, ether and the like), generally in an inert atmosphere (nitrogen or argon) at temperatures in the range of about -78°C to about room temperature, depending, as is well known in the art, on the specific reactive moieties. For example, when a lithio derivative is reacted with a bromide in THF the reaction is conducted at -78°C in an argon atmosphere, whereas when a potassium derivative is reacted with a chloride the reaction is in anhydrous DMF at 70°C. Preferably when cesium is the reactive moiety the condensation is effected in the presence of a crown ether. Once the condensations are effected, the necessary modifications - i.e., deprotection (selective or otherwise) of protected groups, formation of the appropriate leaving groups, de-esterification of the phosphonate esters, and transforming, when desired, the purine and pyrimidine nucleic bases (B') to produce the compounds of Formula **I**.

It is of course obvious that the variety of moieties represented by the partial structure -(CH₂)ₙG-V-D-(CH₂)ₘW-, the components of the specific compound sought to be prepared will dictate the precise steps used in the preparation of both the necessary reactants, particularly those which are to be reacted with compounds **(2)** of Reaction Scheme A. Representative of the methods useful in preparing these reactants (and intermediates therefore) are described in the following illustrations, bearing in mind that these descriptions relate to compounds wherein the nucleic base are not attached directly to a carbon atom bearing an unsaturation.

### ILLUSTRATION I

To prepare compounds of Formula **(3)** wherein W is OCH₂, m is other than zero if D is deleted (or m is as defined generically if D is not deleted) and V, G₂, B and n are as generically defined, the appropriate compound of **(2)** is reacted with Lg-CH₂(P)(O)(OEt)₂ and the leaving group is Br, I, OTos, OMes or OTf and the nucleophile is activated with sodium or potassium.

### ILLUSTRATION II

To prepare compounds of Formula **(3)** wherein W is CFH, CF₂, CCl₂ or CHCl and n, D, V and m are as defined in Illustration I the alcohol of **(2)** is converted to a leaving group (I, Br, OTos, OMes or OTf) and condensed with LiW'P(O)(OEt)₂ with W' being CFH, CF₂, CCl₂ or CHCl.

### ILLUSTRATION III

To prepare compounds of Formula **(3)** wherein W is deleted and n, D, V and m are as defined in Illustration **I** the condensation is better achieved by converted the alcohol **(2)** to a Br or I leaving group and reacting it with a trialkyl-phosphite ester at 140°C using the well known Arburzov reaction.

### ILLUSTRATION IV

To prepare compounds of **(3)** wherein m is zero and W and D are deleted and V is -C(R₃)=C(R₄)- an alkali metal salt of an R₄₋substituted methyl diphosphonate (e.g., M^{⊕} CR₄=((P(O)(OEt)₂)₂ is reacted with a carbonyl derivative (e.g. (Pg(CH₂)ₙG-C(R₃)=O) in anhydrous THF at 20°C followed by quenching with NH₄Cl to produce PgO(CH₂)ₙG-C(R₃)=C(R₄)-P(O)(OEt)₂. This reaction is available whether or not G is deleted. The aldehyde is formed from the corresponding alcohol by oxidation with pyridinium chlorochromate in anhydrous solvent (CH₂Cl₂) in an inert atmosphere at about 20°C. If necessary G may be protected.

### ILLUSTRATION V

In the instance wherein V is -C≡C- and G is also deleted, a lithio derivative of the appropriate phosphonate (LiCH₂P(O)(OEt)₂) (2 equivalents) is condensed with a PgO-(CH₂)ₙC(O)Omethyl) reactant in THF at -78°C under argon for several hours, quenched with saturated aqueous NH₄Cl at about-78°C to-30°C followed by reaction with DAST(diethylaminosulfurtrifluoride) in dichloromethane at about 0°C followed by quenching with excess methanol to produce the desired PgO-(CH₂)ₙC≡C-P(O)(OEt)₂ for use in Step (b) of Reaction Scheme A. When G is not deleted a similar process is used except that its corresponding aldehyde (PgO-(CH₂)ₙCH(R₅)-C(O)(OMethyl) reactant is utilized in place of the aforementioned methyl ester.

### ILLUSTRATION VI

When V is G-C=C=C- and G is either present or deleted, a halide of Formula **(2)** (i.e., PgO-(CH₂)ₙG-C≡C-CH₂Br is reacted with a trialkylphosphite (P(OEt)₃) according to the Arburzov reaction. The so-produced intermediates PgO-(CH₂)ₙG-C≡C-CH₂P(O)(OEt)₂ are subjected to a base catalyzed isomerization to convert acetylene to its corresponding allenyl moiety to produce PgO-(CH₂)ₙG-C=C=C-P(O) (OEt)₂ type compounds ready for Steps (b) and (C) of Reaction Scheme A.

### ILLUSTRATION VII

When V is -C=C-C=C-, again whether or not G is deleted, aldehydes of 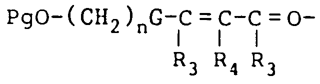
are reacted with an R₄-substituted metallo methylene diphosphonate ester (a sodium salt of tetraethylmethylene diphosphonate, as previously described) and the resulting products 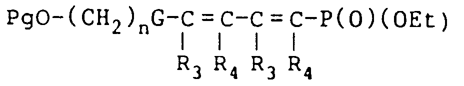
are ready for steps (b) and (c).

Still describing the preparation of the compounds of Formula **(3)** wherein B' is attached to a carbon atom not bearing an unsaturation, three additional special situations are obtained.

### ILLUSTRATION VIII

In the first instance wherein m is zero, W is CF₂ and V is -C(R₃)=(CR₄)- and G and D are deleted, the aldehyde (or ketones) PgO-(CH₂)-C=C(R₃)-C(R₄)=O is reacted with a lithio derivative of a difluoromethylene phosphonate ester to produce a product 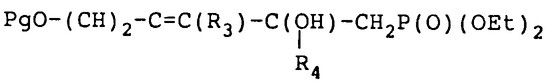
which, upon reaction with thionyl chloride produces compounds 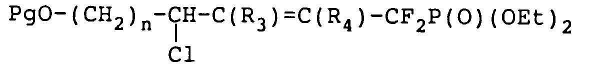
which upon reduction with Zn or Bu₃SnH produces the desired intermediate PgO(CH₂)ₙCH₂C(R₃)=C(R₄)CF₂P(O)(OEt)₂ ready for steps (b) and (c).

### ILLUSTRATION IX

In the second special situation wherein n is one, m is zero, W is CF₂ with V as generically defined, and G and D are deleted, the necessary intermediate of **(4)** is prepared by brominating a compound of formula CH₃-V-CF₂P(O)(OEt)₂ with N-bromosuccinimide (NBS) or other suitable N-bromoamide in a solvent, preferablyCCl₄ to produce BrCH₂-V-CF₂-P(O)(OEt)₂, a compound of Formula **(4)** ready for condensation with a nucleic base. (Of course, N-chlorosuccinimide may be utilized to produce the chloro analogs.)

### ILLUSTRATION X

A third special situation relates to the preparation of a compound of Formula **(4)** wherein V is as generically defined, m and n are one and W is CF₂, and G and D are deleted, entails the reaction of an excess amount of ClCH₂-V-CH₂Cl with a BrZnCF₂P(O)(OEt)₂ in the presence of CuBr in dimethoxyethane to yield ClCH₂VCH₂CF₂P(O)(OEt)₂.

Following the preparation of the compounds of Formula **(3)** by the foregoing described processes, the hydroxy protecting groups are removed and replaced with suitable leaving groups to produce compounds **(4)**, the compounds **(4)** are then condensed with an anion of a purine or pyrimidine nucleic base and the resulting products are de-esterified to remove the ester groups of the phosphonate and, if necessary, make the necessary transformations to form the desired X₁, X₂, X₃, X₄ and X₅ moieties to produce the desired compounds of Formula **I**. These reactions of Steps (b), (c) and (d) of Reaction Scheme A are effected using standard techniques and processes well known in the art and as illustrated in the specific examples herein exemplified.

For the preparation of the compounds of Formula **I**, wherein the nucleic base is attached to a carbon atom bearing an unsaturation, i.e., compounds wherein V is as generically defined, G is deleted and n is zero, there is no one generic scheme applicable to this entire sub-class of compounds, except of course for the de-esterification of the phosphonate ester moiety and, when necessary, the modifications to purine and pyrimidine nucleic bases as defined for the B' moiety. Thus, the preparation of these compounds will be individually described up to the de-esterification/base modifications procedures.

The preparation of compounds of the formula 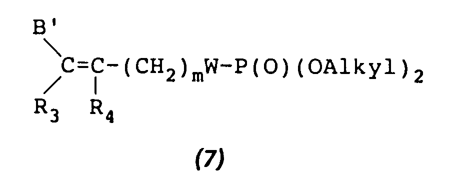
wherein R₃, B', R₄, m and W are as previously defined may be effected by the following reaction scheme. 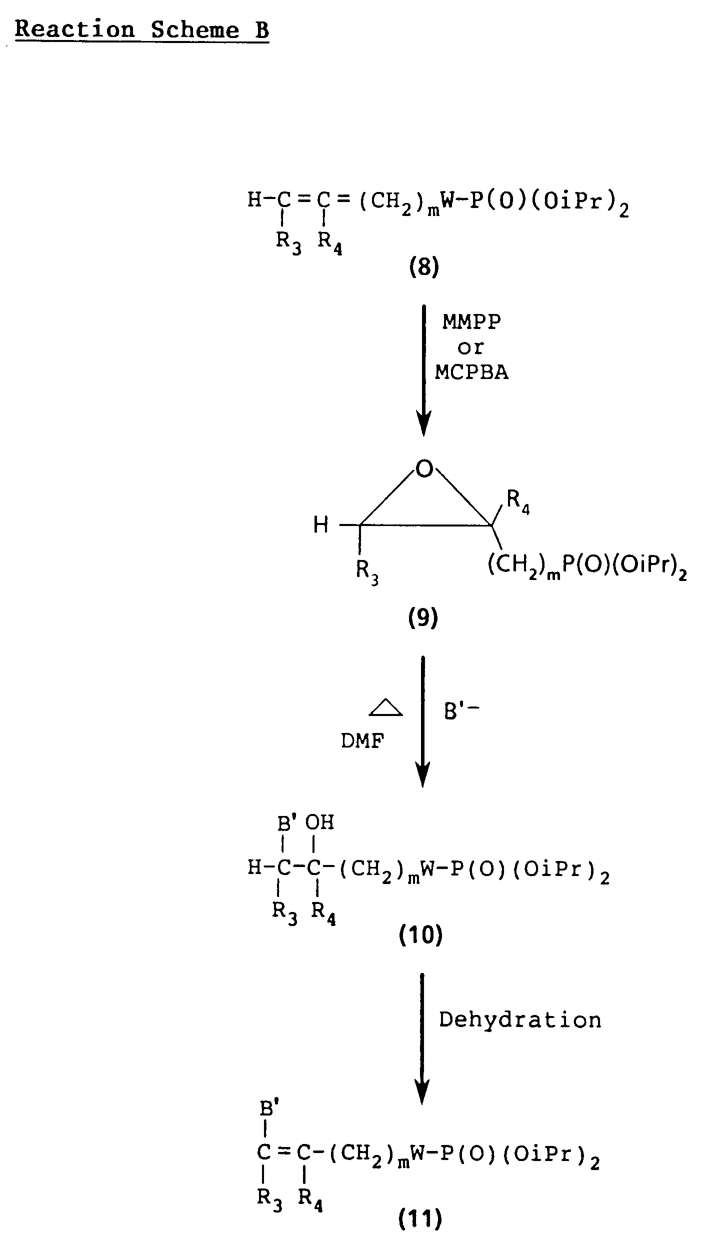
wherein R₃, R₄, m and W are as previously defined. The reaction involves an epoxidation by reaction of compounds **(8)** with meta-chloroperbenzoic acid (MCPBA), or preferably, from a safety point of view, with magnesiummonoperoxyphtalate (MMPA) to obtain the epoxides of **(9)**. The epoxides are condensed with an anionized nucleic base (B') by heating the reactants at about 80-140°C in an anhydrous solvent (DMF) in an inert atmosphere. The nucleic base (B') may be anionized with KH, NaH, K₂CO₃ or Cs₂CO₃ according to standard methodology. The resulting alcohols **(10)** are dehydrated by standard methodology such as, for example, with the use of such agents as thionyl chloride in pyridine, HMPA, or by making the thiocarbonate of the alcohol and then heating the product at about 150°C to produce the compounds **(11)**. The same processes for compounds wherein D is not deleted would be applicable.

The preparation of compounds of the formula 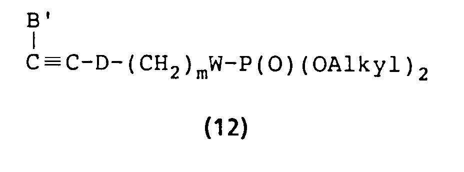
wherein B', D, m and W are as previously defined, may be effected by condensing BrCH₂C(O)D-(CH₂)ₘW-P(O)(OAlkyl)₂ **(13)** with a nucleic base in the presence of a base (e.g., NaH, KH or K₂CO₃) in a non-aqueous solvent using standard procedures. The resulting products B'-CH₂C(O)-D-(CH₂)ₘW-P(O)(OAlkyl)₂ **(14)** are then treated with DAST in triethylamine to produce the desired intermediates of Formula **(12)** ready for de-esterification and any necessary transformation of B' to the desired B compounds of Formula **I**.

The preparation of compounds of the formula 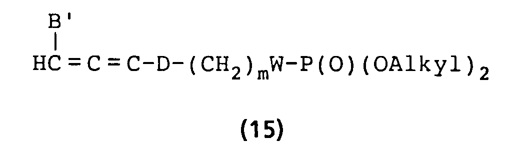
may be accomplished by effecting a basic isomerization on compounds of the formula B'-CH₂C≡C-D-(CH₂)ₘW-P(O)(OAlkyl)₂ **(16)**, the isomerization being effected by reaction with DBU in acetonitrile or DMF, or by a reaction with potassium t-butoxide in t-butanol, or by reaction with LDA in THF. The intermediates **(16)** may be prepared by condensation of the nucleic bases (B') with a compound of the formula Lg-CH₂-C≡C-D-(CH₂)ₘW-P(O)(OAlkyl)₂ **(16)**. In the special instance wherein V is 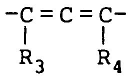
and m is zero (n still being zero), and W is OCH₂, an anionized derivative of B'-HC=C=C-CH₂OH **(17)** is condensed with Lg-CH₂P(O)(OAlkyl)₂ by standard processes to produce B'-HC=C=CHCH₂OCH₂-P(O)(OAlkyl)₂ **(18)**. If W is CF₂, CCl₂, CHF or CHCl then compounds (17) are converted to their tosylate derivatives and then reacted with a lithio derivative (Li-W'-P(O)(OAlkyl)₂ wherein W' is CF₂, CCl₂, CHF or CHCl) to produce compounds B'-HC=C=CH-CH₂-W'P(O)(OAlkyl)₂. If W is deleted then compounds **(17)** are treated with PØ₃Br₂ followed by an Arburzov reaction with P(OEt)₃ to produce compounds B'-HC=C=CHCH₂-P(O)(OAlkyl)₂.

In the instance wherein it is desired to produce compounds of Formula **I** of the formula 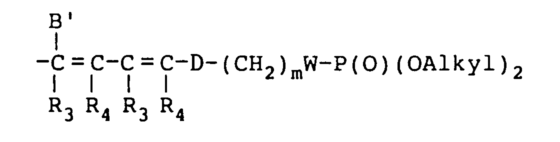
with B', R₃, R₄, D, m and W being as previously defined, compounds of the formula 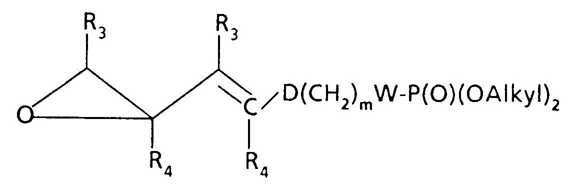
are condensed with an anionized nucleic base (i.e., B') and the resulting products 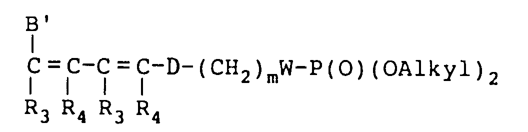
which are subjected to dehydration to produce the intermediates ready for de-esterification of the phosphonate diester and for any desired transformation of the nucleic base.

Further, for preparing compounds wherein V is still 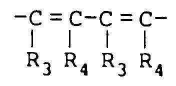
but in the special instance wherein m is zero, and D and W are deleted, i.e., compounds of the formula 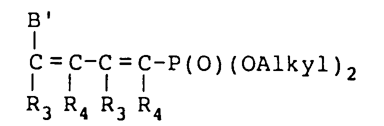
may be produced by treating compounds 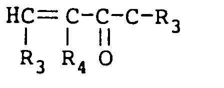
with 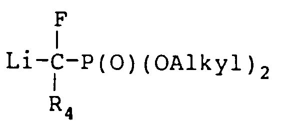
to produce compounds 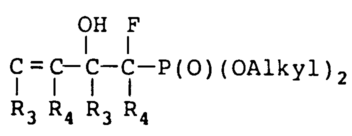
which, when subjected to a rearrangement reaction with thionyl chloride, produce compounds 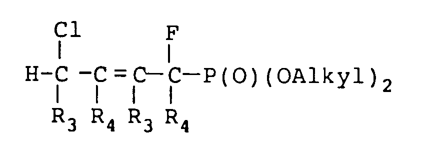
which, when condensed with the nucleic base in the presence of Cs₂CO₃ produces the desired intermediate ready for de-esterification and nucleic base modifications, if desired, to produce the desired compounds of Formula **I**.

The following examples will illustrate the methods by which the compounds of this invention may be prepared.

### EXAMPLE 1

### [5-(2-Amino-1,6-dihydro-6-oxo-9H-purin-9-yl)-1,1-difluoro-penten-3-yl] phosphonic acid

### Step A: 9-[4-Chloro-2-buten-1-yl]-2-amino-6-chloropurine

4.8 g of 60% NaH in oil are slowly added to a stirred suspension of 100 mmol (17 g) of 2-amino-6-chloro-purine in 100 ml of anhydrous dimethylformamide at 20°C under argon. After stirring for 30 minutes at 20°C, 300 mmol (31.5 g) of 1,4-dichloro-2-butene in 100 ml of dimethylformamide are added and the mixture is stirred at 20°C for 18 hours, evaporated to dryness and the expected product is obtained in a pure form (13 g, 50% yield) after flash chromatography on silica gel.

### Step B: [5-(2-Amino-6-chloro-9H-purin-9-yl)-1,1-difluoro-penten-3-yl] phosphonic acid, diethyl ester

15 mmol of diethylbromodifluoromethane phosphonate (3.99 g) are slowly added to a stirred suspension of zinc dust (0.975 g) in 8 ml of dimethoxyethane. After stirring for 8 hours at 20°C, the reaction mixture is filtered to remove excess zinc and added to a stirred solution of 9-[4-chloro-2-buten-1-yl]-2-amino-6-chloropurine (5 mmol) in 10 ml of anhydrous dimethoxyethane containing 10 g of copper (I) bromide. The reaction mixture is stirred in the dark for 7 hours, evaporated to dryness and purified by flash chromatography on silica gel to give 1.17 g of the expected compound [5-(2-amino-6-chloro-9H-purin-9-yl)-1,1-difluoro-penten-3-yl] phosphonic acid, diethyl ester (58% yield).

### Step C: [5-(2-Amino-1,6-dihydro-6-oxo-9H-purin-9-yl)-1,1-difluoro-penten-3-yl] phosphonic acid

1 ml of trimethylsilylbromide is added to a stirred solution of A₂ (1.6 mmol, 0.64 g) in 7.5 ml of dichloromethane at 20°C under argon. The reaction mixture is stirred at 20°C for 20 hours, evaporated under reduced pressure and quenched with water in acetonitrile. The white precipitate is collected, dissolved in aqueous 1N HCl (10 ml) and 2 ml of tetrahydrofuran and heated at 100°C for 18 hours. The final product which precipitates on cooling is collected and dried to give 1 mmol of the title compound [5-(2-amino-1,6-dihydro-6-oxo-9H-purin-9-yl)-1,1-difluoro-penten-3-yl] phosphonic acid.
¹H NMR (360 MHz, D₂O/NaOD) δ 7.7 (s, 1H), 5.85 (m, 2H), 4.8 (m, 5H), 4.7 (d, 2H), 3 (dt, 2H); (homonuclear decoupling shows J_{H2-H3} = 11 Hz).
¹⁹F NMR (360 MHz, D₂O/NaOD) δ - 35.8 ppm (dt, JF-P = 84 Hz). Anal. calcd for C₁₀H₁₂F₂N₅O₄P, H₂O: C, 34.00; H, 4.00; N, 19.83. Found: C, 33.71; H, 4.08; N, 19.34.

### EXAMPLE 2

### [5-(1,6-Dihydro-6-oxo-9H-purin-9-yl)-1,1-difluoro-penten-3-yl] phosphonic acid

### Step A: 9-[4-Chloro-2-buten-1-yl]-6-chloropurine

4.8 g of 60% NaH in oil are slowly added to a stirred suspension of 100 mmol (17 g) of 6-chloro-purine in 100 ml of anhydrous dimethylformamide at 20°C under argon. After stirring for 30 minutes at 20°C, 300 mmol (31.5 g) of 1,4-dichloro-2-butene in 100 ml of dimethylformamide are added and the mixture is stirred at 20°C for 18 hours, evaporated to dryness and the expected product is obtained in a pure form (13 g, 50% yield) after flash chromatography on silica gel.

### Step B: [5-(6-Chloro-9H-purin-9-yl)-1,1-difluoro-penten-3-yl] phosphonic acid, diethyl ester

The expected product is obtained by following the same procedure as used in the preparation of Example 1, Step B.

### Step C: [5-(1,6-Dihydro-6-oxo-9H-purin-9-yl)-1,1-difluoro-penten-3-yl] phosphonic acid

The title compound is obtained by following the same procedure as used in the preparation of Example 1, Step C. ¹H NMR (360 MHz, D₂O) δ 8.15 (s, 1H), 7.95 (s, 1H), 5.9 (m, 2H), 4.85 (d, 2H), 4.75 (m, 3H), 3 (dt, 2H).
¹⁹F NMR (360 MHz, D₂O/NaOH) δ - 35.75 (dt, JH-F = 20 Hz, JF-P = 72 Hz).
Anal. calcd for C₁₀H₁₂F₂N₄O₄P, HBr, 1.25 H₂O: C, 28.35; H, 3.45; N, 13.23. Found: C, 28.34; H, 3.70; N, 12.82.

### EXAMPLE 3

### [5-(6-Amino-9H-purin-9-yl)-1,1-difluoro-penten-3-yl] phosphonic acid

0.84 g (2.1 mmol) of [5-(1,6-dihydro-6-chloro-9H-purin-9-yl)-1,1-difluoro-penten-3-yl] phosphonic acid, diethyl ester (prepared as described in Example 2, Step B) is dissolved in 100 ml of methanol saturated with anhydrous ammonia. The mixture is heated at 100°C in a steel bomb for 24 hours, then cooled to 0°C and evaporated to dryness. The residue is dissolved in 25 ml of anhydrous dichloromethane and treated with 0.25 ml of trimethylsilylbromide at 20°C under argon. The reaction mixture is stirred at 20°C for 20 hours, evaporated, dissolved in 5 ml of acetonitrile and quenched with 0.5 ml of water. The title compound [5-(6-amino-9H-purin-9-yl)-1,1-difluoro-penten-3-yl] phosphonic acid is obtained after recrystallization from water. ¹H NMR (360 MHz, D₂O/NaOD) δ 8.2 (s, 1H), 8.1 (s, 1H), 5.95 (m, 2H), 4.95 (d, 2H), 4.85 (m, 4H), 3 (dt, 2H); (homonuclear decoupling shows J_{H2-H3} = 11 Hz).
¹⁹F NMR (360MHz, D₂O/NaOD) δ - 35.8 (dt, JH-F = 20 Hz, JF-P = 90).
Anal. calcd for C₁₀H₁₂F₂N₅O₄P, 1.5 H₂O; 0.25 HBr: C, 32.78; H, 4.19; N, 19.11. Found: C, 32.97; H, 4.47; N, 18.58.

### EXAMPLE 4

### [4-(6-Amino-9H-purin-9-yl)-1,1-difluoro-2-butenyl] phosphonic acid

### Step A: (1,1-Difluoro-2-hydroxy-buten-3-yl) phosphonic acid, diethyl ester

50 mmol of (diethyl phosphinyl) difluoromethane (9.4 g) dissolved in 50 ml of anhydrous tetrahydrofuran are slowly added to a stirred solution of lithium diisopropylamine (freshly prepared from 50 mol of n-butyllithium and 50 mmol of diisopropylamine in 50 ml of tetrahydrofuran) at -78°C under argon. After stirring for 30 minutes, acrolein (60 mmol) dissolved in 20 ml of tetrahydrofuran is added to the reaction mixture which is stirred at -78°C for 3 hours, quenched at -78°C with excess saturated aqueous ammonium chloride and evaporated to dryness. Usual work-up and flash chromatography purification gives the expected product 1,1-difluoro-2-hydroxy-buten-3-yl) phosphonic acid, diethyl ester.

### Step B: (1,1-Difluoro-4-chloro-buten-2-yl) phosphonic acid, diethyl ester

24 mmol of freshly distilled thionyl chloride (1.76 ml) are added to a stirred solution of 20 mmol of 1,1-difluoro-2-hydroxy-buten-3-yl) phosphonic acid, diethyl ester (4.77 g) in 60 ml of anhydrous dichloromethane. The reaction mixture is stirred at refluxing temperature and excess thionyl chloride (0.8 ml) is added after 18 hours. After 40 hours at 40°C, the reaction mixture is evaporated and purified by flash chromatography on silica gel giving 3.79 g of the expected product (1,1-difluoro-4-chloro-buten-2-yl) phosphonic acid, diethyl ester (76% yield).

### Step C: [4-(6-Amino-9H-purin-9-yl)-1,1-difluoro-2-butenyl] phosphonic acid, diethyl ester

23 mmol of potassium fluoride (1.34 g) are added to a stirred suspension of 4.2 mmol of adenine (0.57 g) and 4.2 mmol of (1,1-difluoro-4-chloro-buten-2-yl) phosphonic acid, diethyl ester (1.1 g) in 11 ml of anhydrous dimethylformamide. The reaction mixture is stirred at 50°C for 4 hours and at 20°C for 20 hours, evaporated under reduced pressure and purified by flash chromatography on silica gel to give 0.58 g of the expected product [4-(6-amino-9H-purin-9-yl)-1,1-difluoro-2-butenyl] phosphonic acid, diethyl ester (26% yield).

### Step D: [4-(6-Amino-9H-purin-9-yl)-1,1-difluoro-2-butenyl] phosphonic acid

0.7 ml of trimethylsilylbromide is added to a stirred solution of [4-(6-amino-9H-purin-9-yl)-1,1-difluoro-2-butenyl] phosphonic acid, diethyl ester (1.2 mmol, 0.43 g) in 6 ml of anhydrous dichloromethane at 20°C under argon. The reaction mixture is evaporated to dryness, dissolved in 2.8 l of acetonitrile and quenched with water. The title compound [4-(6-amino-9H-purin-9-yl)-1,1-difluoro-2-butenyl] phosphonic acid is obtained after recrystallization from water (0.32 g, 28% yield).
¹H NMR (360 MHz, D₂O/NaOD) δ 8.2 (s, 1H), 8.1 (s, 1H), 6.35 (dt, 1H), 5.8 (dt, 1H, JH-H = 16 Hz), 5 (D, 2H), 4.8 (m, 4H). ¹⁹F NMR (360MHz, D₂O/NaOD) δ - 32.8 (dd, JH-F = 22 Hz, JF-P = 88 Hz).
Anal. calcd for C₉H₁₀F₂N₅O₃P, 0.5 HBr, 2H₂O: C, 28.32; H, 3.83; N, 18.35. Found: C, 28.07; H, 3.64; N, 18.02.

### EXAMPLE 5

### [4-(6-Amino-9H-purin-9-yl)-1-fluoro-1,3-butadienyl] phosphonic acid

### Step A: [4-(6-Amino-9H-purin-9-yl)-1-fluoro-1,3-butadienyl] phosphonic acid, diethyl ester

13 mmol of cesium carbonate (4.24 g) are added in one portion to a stirred suspension of 3.5 mmol of (1,1-difluoro-4-chloro-buten-2-yl) phosphonic acid, diethyl ester and 3.5 mmol of adenine hydrochloride in 13 ml of anhydrous dimethylformamide. The reaction mixture is stirred at 20°C for 20 hours, evaporated to dryness and purified by flash chromatography on silica gel to give 0.43 g of the expected product [4-(6-amino-9H-purin-9-yl)-1-fluoro-1,3-butadienyl] phosphonic acid, diethyl ester (34% yield).

### Step B: [4-(6-Amino-9H-purin-9-yl)-1-fluoro-1,3-butadienyl] phosphonic acid

0.7 ml of trimethylsilylbromide is added to a solution of 0.43 g of 4-[(6-amino-9H-purin-9-yl)-1-fluoro-1,3-butadienyl] phosphonic acid, diethyl ester in 6 ml of anhydrous dichloromethane at 20°C under argon. The reaction mixture is stirred at 20°C for 20 hours and evaporated to dryness. The residue is dissolved in 3 ml of acetonitrile, quenched with water and evaporated. The residue is dissolved in hot ethanol and crystallized on cooling to give 0.212 g of the title compound [4-(6-Amino-9H-purin-9-yl)-1-fluoro-1,3-butadienyl] phosphonic acid (58% yield).
¹H NMR (360 MHz, D₂O/NaOD) δ 8.2 (s, 1H), 8.10 (s, 1H), 6.65 (m, 2H), 6.2 ppm (m, 1H).
¹⁹F NMR (360MHz, D₂O/NaOD) δ - 37.25 (d, JF-P = 87 Hz). Anal. calcd for C₉H₉FN₅O₃P, H₂O: C, 35.65; H, 3.66; N, 23.10. Found: C, 35.76; H, 3.63; N, 22.79.

### EXAMPLE 6

### [3-[(6-Amino-9H-purin-9-yl)methyl]-1,1-difluoro-3-butenyl] phosphonic acid

### Step A: [3-Methyl-1,1-difluoro-3-butenyl] phosphonic acid, diethyl ester

30 mmol (7.98 g) of (diethyl phosphinyl)difluorobromomethane are added to a stirred suspension of zinc (1.9 g) in 25 ml of anhydrous dimethoxyethane at 20°C under argon. After 3 hours at 20°C, the reaction mixture is filtered to remove the unreacted zinc (0.465 g) and metallylchloride (2.3 ml) is added to the solution together with 0.2 g of copper (I) bromide. The reaction mixture is stirred in the dark for 20 hours, evaporated under reduced pressure and purified by flash chromatography on silica gel to give 3.3 g of the expected product [3-methyl-1,1-difluoro-3-butenyl] phosphonic acid, diethyl ester (64% yield).

### Step B: [3-[(6-Amino-9H-purin-9-yl)methyl]-1,1-difluoro-3-butenyl] phosphonic acid, diethyl ester

9 mmol of N-bromo-succinimide and a few milligrams of benzoyl peroxide are added to a solution of 3-methyl-1,1-difluoro-3-butenyl phosphonic acid, diethyl ester (9 mmol) in 18 ml of carbon tetrachloride. The suspension is heated at refluxing temperature with a lamp until all solid is floating up. The allylic bromide is then isolated after filtration and evaporation of the solvent is an oil which is added to a stirred suspension of 9 mmol of adenine and 18 mmol of potassium carbonate in 20 ml of anhydrous dimethylformamide at 20°C under argon. The reaction mixture is stirred at 20°C for 20 hours, evaporated under reduced pressure and purified by flash chromatography on silica gel to give 1.38 g of the expected product [3-[(6-amino-9H-purin-9-yl)methyl]-1,1-difluoro-3-butenyl] phosphonic acid, diethyl ester (40% yield).

Step C:
[3-[(6-Amino-9H-purin-9-yl)methyl]-1,1-difluoro-3-butenyl] phosphonic acid,

The title compound is obtained from [3-[(6-amino-9H-purin-9-yl)methyl]-1,1-difluoro-3-butenyl] phosphonic acid, diethyl ester by reaction with trimethylsilylbromide, as previously described for the transformation of [4-(6-amino-9H-purin-9-yl)-1-fluoro-1,3-butadienyl] phosphonic acid, diethyl ester into [4-(6-amino-9H-purin-9-yl)-1-fluoro-1,3-butadienyl] phosphonic acid.
¹H NMR (360 MHz, D₂O/NaOD) δ 8.25 (s, 1H), 8.2 (s, 1H), 5.2 (s, 1H), 4.95 (s, 2H), 4.7 (s, 1H), 2.95 (t, 2H). ¹⁹F NMR (360MHz, D₂O/NaOD) δ - 35.6 (dt, JF-H = 21 Hz), JF-P = 85 Hz).
Anal. calcd for C₁₀H₁₂F₂N₅O₃P, H₂O: C, 35.62; H, 4.18; N, 20.77. Found: C, 35.95; H, 4.27; N, 20.64.

### EXAMPLE 7

### [5-(6-Amino-9H-purin-9-yl)-1-pentenyl] phosphonic acid

### Step A: 5-(6-Amino-9H-purin-9-yl)-1-pentene

27.72 g of cesium carbonate (85 mmol) are added to a stirred suspension of adenine (5 g, 37 mmol) in 80 ml of anhydrous dimethylformamide at 20°C under argon. After 30 minutes, 5-bromo-1-pentene (7.21 g, 48 mmol) is added to the reaction mixture which is stirred at 20°C for 20 hours, evaporated under reduced pressure and purified by flash chromatography on silica gel to give 5.34 g of the expected product 5-(6-amino-9H-purin-9-yl)-1-pentene (71% yield).

### Step B: 5-[(6-(Bisbenzoyl)amino-9H-purin-9-yl)]-1-pentene

Benzoyl chloride (14.05 g, 100 mmol) and 14 ml of triethylamine are successively added to a stirred solution of 8.6 g (42.5 mmol) of 5-(6-amino-9H-purin-9-yl)-1-pentene, dissolved in 100 ml of anhydrous dichloromethane at 20°C under argon. After 6 hours, 0.45 g of 4-dimethylaminopyridine are added to the reaction mixture which is stirred at 45°C for 15 hours. The crude mixture is washed with water, bicarbonate and brine, evaporated under reduced pressure and purified by flash chromatography on silica gel to give 14.8 g of the expected product 5-[(6-(bisbenzoyl)-amino-9H-purin-9-yl)]-1-pentene (85% yield).

### Step C: [5-[(6-(Bisbenzoyl)amino-9H-purin-9-yl)]-1-pentenyl] phosphonic acid, diethyl ester

Ozone (10 mmol) is bubbled through a solution of 5-[(6-(bisbenzoyl)amino-9H-purin-9-yl)]-1-pentene (2.52 g, 6.13 mmol) in 6 ml of dichloromethane and 3 ml of methanol, at -78°C. After 15 minutes, excess ozone is removed by bubbling nitrogen to the solution at -78°C, and 6.5 mmol of dimethyl sulfide are added to the reaction mixture which is slowly stirred to 20°C. The crude mixture is evaporated under reduced pressure and purified by flash chromatography on silica gel to give 1.47 g of aldehyde (66%) which is suspended in 5 ml of tetrahydrofuran and added to a stirred solution of the lithium salt of tetraethyl methylene bisphosphonate (prepared by adding 5.3 mmol of n-butyllithium to 5.3 mmol of tetraethylmethylene bisphosphonate on 8 ml of tetrahydrofuran at -78°C under argon). The reaction mixture is stirred at -78°C for 3 hours, at 20°C for 5 hours, quenched with saturated aqueous ammonium chloride and evaporated. The residue is extracted with ethyl acetate (5 × 30 ml). The organic layers are evaporated and purified by flash chromatography on silica gel to give 1.37 g of the expected product [5-[(6-(bisbenzoyl)amino-9H-purin-9-yl)]-1-pentenyl] phosphonic acid, diethyl ester.

### Step D: [5-(6-Amino-9H-purin-9-yl)-1-pentenyl] phosphonic acid

Sodium methoxide (0.1 g) is added to a stirred solution of 0.92 g (1.68 mmol) of [5-[(6-(bisbenzoyl)amino-9H-purin-9-yl)]-1-pentenyl phosphonic acid, diethyl ester dissolved in 5 ml of methanol at 20°C. After stirring for 20 hours, the reaction mixture is evaporated and purified by flash chromatography on silica gel to give 0.47 g (83%) of [5-(6-amino-9H-purin-9-yl)-1-pentenyl] phosphonic acid, diethyl ester. This compound is dissolved in 5 ml of anhydrous acetonitrile and heated by 0.7 ml (5.4 mmol) of trimethylsilylbromide at 20°C under argon. After stirring for 18 hours at 20°C, the reaction mixture is evaporated under reduced pressure. The residue is dissolved in acetonitrile and precipitates upon addition of water. Recrystallization from ethanol gives 0.65 g of the title compound [5-(6-amino-9H-purin-9-yl)-1-pentenyl] phosphonic acid.

### EXAMPLE 8

### [5-(2-Amino-1,6-dihydro-6-oxo-9H-purin-9-yl)-1-pentenyl] phosphonic acid

### Step A: 4⁻(^{t}Butyldimethylsilyloxy)-1-butanol

^{t}Butyldimethylsilyl chloride (25 g, 165 mmol) dissolved in 60 ml of anhydrous dichloromethane is added to a stirred solution of 13.6 g (150 mmol)) of 1,4-butanediol, 25 ml of triethylamine and 2 g of 4-dimethyl aminopyridine in 300 ml of dichloromethane. The reaction mixture is stirred at 20°C for 20 hours, washed with aqueous saturated ammonium chloride, bicarbonate and brine, dried, evaporated and purified by flash chromatography on silica gel to give 18.1 g of the expected product 4-(^{t}butyldimethylsilyloxy)-1-butanol (59% yield).

### Step B: [5-(^{t}Butyldimethylsilyloxy)-1-pentenyl] phosphonic acid, diethyl ester

4-(^{t}butyldimethylsilyloxy)-1-butanol (18.1 g, 89 mmol) dissolved in 25 ml of dichloromethane is added dropwise to a vigorously stirred suspension of pyridinium chlorochromate (20.7 g) in 100 ml of dichloromethane. After 1½ hours, 200 ml of ether are added and the mixture is stirred for 1½ hours. The mixture is filtered over florisil and evaporated to give 13 g of crude product which is distilled (48°C/0.35 mmHg) to give 7.4 g of 4-(^{t}butyldimethylsilyloxy)-1-butenal (42% yield). This aldehyde is added to the lithium salt of tetraethylmethylenebisphosphonate (prepared by adding 50 mmol of n-butyllithium to a solution of 50 mmol of tetraethylmethylenebisphosphonate in 60 ml of tetrahydrofuran at -78°C). The reaction mixture is stirred at -78°C for 3 hours, at 20°C for 20 hours, quenched with aqueous saturated ammonium chloride and evaporated under reduced pressure. The residue is extracted with ethyl acetate (5 × 60 ml), evaporated and purified by flash chromatography on silica gel to give 11.07 g of the expected product [5-(^{t}butyldimethylsilyloxy)-1-pentenyl] phosphonic acid, diethyl ester (91% yield).

### Step C: (5-Tosyloxy-1-pentenyl) phosphonic acid, diethyl ester

Trihydrated tetrabutylammonium fluoride (5.63 g, 18 mmol) is added to a stirred solution of [5-(^{t}butyldimethylsilyloxy)-1-pentenyl] phosphonic acid, diethyl ester (4 g, 12 mmol) in 25 ml of tetrahydrofuran at 20°C. After 2 hours, the reaction mixture is evaporated; the residue is dissolved in ethyl acetate, washed with water and bicarbonate, dried over sodium sulfate, filtered, evaporated and purified by flash chromatography on silica gel to give 2.27 g (86%) of the expected product which is dissolved in 0.15 g of anhydrous dichloromethane and treated successively by 1.6 ml of triethylamine, 0.15 g of 4-dimethylamino pyridine and 2.15 g of tosyl chloride at -10°C under argon. The reaction mixture is stirred at 20°C for 20 hours, evaporated and purified by flash chromatography on silica gel to give 2.72 g of the expected product (5-tosyloxy-1-pentenyl) phosphonic acid, diethyl ester(72% yield).

### Step D: [5-(2-Amino-6-chloro-9H-purin-9-yl)-1-pentenyl] phosphonic acid, diethyl ester

1.35 g (7.96 mmol) of 2-amino-6-chloro purine is added to a stirred suspension of sodium hydride (8 mmol) in 8 ml of dimethylformamide at 20°C. After 30 minutes, the intermediate (5-tosyloxy-1-pentenyl) phosphonic acid, diethyl ester (2.72 g, 7.23 mmol) dissolved in 7 ml of dimethylformamide is added to the reaction mixture which is stirred at 60°C for 20 hours, evaporated under reduced pressure and purified by flash chromatography on silica gel to give 1.5 g of the expected product [5-(2-amino-6-chloro-9H-purin-9-yl)-1-pentenyl] phosphonic acid, diethyl ester (56%) yield).

### Step E: [5-(2-Amino-1,6-dihydro-6-oxo-9H-purin-9-yl)-1-pentenyl] phosphonic acid

The title compound is obtained from [5-(2-amino-6-chloro-9H-purin-9-yl)-1-pentenyl] phosphonic acid, diethyl ester by following the two-step procedure described for the preparation of Example 1, Step C.

Purine nucleoside phosphorylase (PNP) under normal *in vivo* conditions catalyzes the phosphorolytic cleavage of the ribo- and deoxyribonucleosides of guanine and hypoxanthine to the corresponding sugar phosphate and guanine or hypoxanthine. In the absence of PNP, uric acid concentration is quite low while the concentration of certain nucleoside substrates of PNP such as (dGuo) in plasma and urine are elevated. dGuo is toxic towards lymphoblasts, however, T-cells are much more affected than are B-cells. Indeed, in patients with genetically acquired PNP deficiency, B-cell immunoglobulin production is normal or even elevated, but these patients are leukopenic and T-lymphocytic function is either totally lacking or is severely depressed. While uncontrolled PNP deficiency is obviously undesirable, there are many instances where controlled suppression of the immune system, and in particular controlled supression of T-cells, would be highly desirable such as in the treatment of T-cell leukemia, the suppresion of host-vs-graft response in organ transplant recipients, and the treatment of gout.

The compounds of this invention are also useful in the medical therapy particularly for the treatment or prophylaxis of viral infections such as for example hepatitis B, retroviral infections, especially AIDS, respiratory syncitial viruses, and herpes viruses such as herpes simplex I and II, cytomegalovirus and varicella-zoster-virus.

Examples of retroviral infections which may be treated or prevented in accordance with the invention include human retroviral infections such as Human Immunodeficiency Virus (HIV), HIV-2 and Human T-cell Lymphotropic Virus (HLTV) e.g. HTLV-I or HTLV-IV infections. The compounds according to the invention are especially useful for the treatment or prophylaxis of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura. The compounds may also be used in the treatment or prevention of psoriasis.

In a further aspect of the present invention there is included: a) a method for the treatment and prophylaxis of retroviral infections and hepatitis B infections which comprises treating the patient with a therapeutically effective amount of a compound of this invention; b) use of a compound of this invention in the manufacture of a medicament for the treatment or prophylaxis of any of the herein-mentioned infections or conditions.

The ability of the compounds of this invention to act as immunosuppressant, antilymphoma, antileukemic, antiviral, and antiprotozoal agents and as agents useful in the treatment of gout, psoriasis and autoimmune diseases can be assessed using standard laboratory *in vitro* and *in vivo* methodology including their ability to inhibit purine nucleoside phosphorylase (PNP). Purine nucleoside phosphorylase (PNP) inhibitory activity can be determined by the coupled xanthine oxidase method of Kalckar, using inosine as the substrate [H.M. Kalckar, J. Biol. Chem. 167, 429-443 (1947)]. Apparent dissociation constants (K_{I}) were measured at 1 mM inorganic phosphate using 0.1 M HEPES buffer (pH 7.4), four concentrations of inosine ranging from 0.05 mM to 0.15 mM and various concentrations of inhibitor. The Kᵢ for representative members of the compounds of formula 1 are tabulated in table 1 and are compared to the K_{M} values of the substrate inosine using PNP from various sources. Moreover, compounds of this invention have been shown to be effective against lymphomas (human MoLT-4 cells) and thus have antilymphomic and antileukemic activities. The presence of 2'-deoxyguanosine (about 1-10 µM), a natural metabolite, appears to be important for activity against lymphoma cells in culture.

The amount of the active ingredient to be administered can vary widely according to the particular dosage unit employed, the period of treatment, the age and sex of the patient treated and the nature and extent of the disorder treated. The total amount of the active ingredient to be administered will generally range from about 1 mg/kg to 100 mg/kg and preferably from 3 mg/kg to 25 mg/kg. A unit dosage may contain from 25 to 500 mg of active ingredient, and can be taken one or more times per day. The active compound of formula 1 can be administered with a pharmaceutical carrier using conventional dosage unit forms either orally, parenterally, topically or transdermally.

As used herein the term "patient" includes mammals such as mice, rats, cats, dogs, cattle, sheep, swine, and primates including humans. In regard to the treatment of parasitic infections includes not only mammals but also other warm blood animals such as fowl including chickens and turkey.

The term protozoa is intended to include those members of the subphyla *Sarcomastigophora* and *Sprozoa* of the phylum *Protozoa*. More particularly the term protozoa as used herein is intended to include those genera of parasitic protozoa which are important to man because they either cause disease in man or in his domestic animals. These genera are for the most part found classified in the superclass of *Mastigophora* of the subphylum *Sarcomastigophora* and the class of *Telosporea* of the subphylum *Sporozoa* in the classification according to Baker (1969). Illustrative genera of these parasitic protozoa include *Histomonas*, *Trypanosoma*, *Giardia*, *Trichomonas*, *Eimeria*, *Isopora*, *Toxoplasma*, and *Plasmodium*.

Included as an embodiment of the present invention is the use of these compounds as antiprotozoal agents in the treatment of intestinal coccidia in commercial poultry. Intestinal coccidia infections are responsible for multi-million dollars loses to the poultry industry in the United States each year. Due to the rapid development of drug resistance by coccidia, and due to the relatively high toxicity of some of the drugs used in the treatment of coccidiosis, there is a need for effective coccidiostats that are non-toxic and to which intestinal coccidia do not develop rapid drug resistance.

For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutical adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The phrmaceutically effective compound dissolves in and diffuses through the matrix itself. Release by microporous flow occurs when the pharmaceutically effective compound is transported through a liquid phase in the pores of the matrix.

The compounds of this invention may also be employed in combination with other therapeutic agents for the treatment or prophylaxis of the above infections or conditions. Examples of such further therapeutic agents include agents that are effective for the treatment or prophylaxis of viral infections or associated conditions such as 3'-azido-3'-deoxythymidine (zidovudine), 2',3'-dideoxynucleosides such as 2',3'-dideoxycytidine, 2',3'-dideoxy adenosine and 2',3'-dideoxyinosine, acyclic nucleosides (e.g. acyclovir), interferons such as α-interferon, renal excretion inhibitors such as probenicid, nucleoside transport inhibitors such as dipyridamole, as well as immunomodulators such as interleukin II and granulocyte macrophage colony stimulating factors. The component compounds of such combination therapy may be administered simultaneously, in either separate or combined formulations, or at different times, e.g. sequentially such that a combined effect is achieved.

Another aspect of this invention is the use of the purine nucleoside phosphorylase inhibitors of formula I in conjunctive therapy to potentiate the efficacy of antiviral nucleoside analogs which would otherwise become subject to the enzymatic action of purine nucleoside phosphorylase.

It is known that purine nucleoside derivatives administered as antiviral agents are subject to a purine nucleoside phosphorylase catalysis which undesirably alters the efficacy of such agents. Indeed, the catalysis may lead to untoward side effects. It is also known that antiviral compounds which, per se, are not subject to purine nucleoside phosphorylase will, (either by mechanisms well understood e.g., enzymatic action of adenosine deaminase or by mechanisms not understood) become subject to the action of purine nucleoside phosphorylase, and thus the antiviral effectiveness of this type of compound will similarly be altered. Thus, for the purpose of this aspect of the invention both types of antiviral agents are embraced by the term antiviral agents subject to purine nucleoside phosphorylase.

In particular, this invention embraces the use of a compound of Formula I in conjunctive therapy for the treatment of retroviral infections, especially in humans, particularly human immunodeficiency virus. Particularly preferred 2',3'-dideoxy purine nucleosides are 2'3'-dideoxy-adenosine, 2',3'-dideoxyguanosine, 2',3'-dideoxythioinosine and 2',3'-dideoxyinosine.

The potentiation of the anti-retroviral effect of a dideoxypurine nucleoside, e.g. of Formula I, by a PNP inhibitor can be determined, e.g. in cell cultures (e.g. H9 cells, ATH8 cells) exposed to a retrovirus (e.g. HIV) according to methodology well known in the art, such as described in Proc. Nat. Acad. Sci, U.S.A., 83, 1911 (1986). The potentiation can also be ddetermined *in vivo* (e.g. in rats) by measuring the increase in the plasma level of the dideoxypurine nucleoside which is achieved by prior or simultaneous administration of the particular PNP inhibitor, according to methodology well known in the art.

The two active ingredients (2',3'-dideoxypurine nucleoside and PNP inhibitor) may be administered simultaneously by the same or different routes, in the same or different formulations or may be administered at discrete points in time provided that there is effective PNP inhibition when the 2',3'-dideoxypurine nucleoside is present. The extent to which this time separation of administered active agents can be accomplished depends upon the amount of available PNP and the rate at which the PNP inhibitor is itself degraded. For these reasons, the preferred dosage is in divided doses two to four times a day, most preferably with both agents being administered simultaneously.

This aspect of the invention may alternatively be expressed as: in the process for treating viral infections with antiviral agents subject to nucleoside phosphorylase, the improvement which comprises conjunctively administering a therapeutically effective amount of a purine nucleoside phosphorylase inhibitor, particularly those inhibitors embraced herein by those compounds embraced within the scope of the generic scope of Formula **I**.

In this aspect of the invention, the term "antiviral" includes the treatment of viruses and diseases caused thereby - commonly known to be amenable to treatment with nucleoside analogs such as, for example, the HIV viruses imputed to be causative factors in AIDS, hepatitis B virus and herpes.

Particular antiviral agents of current interest for which enhanced antiviral efficacy would be achieved with conjunctive therapy with the PNPase inhibitors are such compounds as
(a) dideoxy nucleosides of the formula 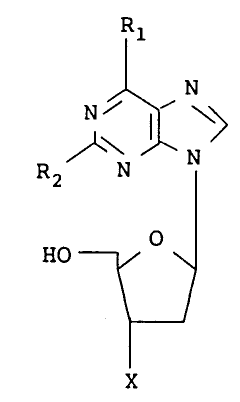 wherein

| **R₁, R₂, X** | **Name** | **Target Virus** |
|---|---|---|
| OH, H, H | dideoxyinosine | HIV |
| OH, NH₂, H | dideoxyguanosine | HIV, |
| | | Hepatitis B |
| OH, NH₂, F | 3'-F-dideoxyguanosine | HIV |
| OH, NH₂, N₃ | 3'-azidodideoxyguanosine | HIV, |
| | | Hepatitis B |
| NH₂, NH₂, H | dideoxydiamino purine riboside | HIV, HBV |
| NH₂, H, H | dideoxyadenosine | HIV |
| NH₂, NH₂, N₃ | 3'-azidodideoxydiamino-purine riboside | HIV, HBV |

The last three compounds are first substrates of adenosine deaminase,
(b) dideoxy dehydro nucleosides of the formula 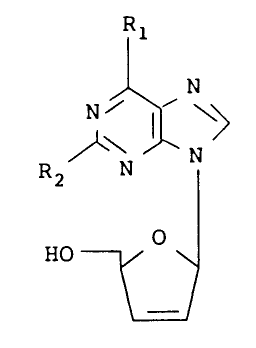 wherein

| **R₁, R₂** | **Name** | **Target virus** |
|---|---|---|
| OH, H | dideoxydehydro inosine | HIV |
| OH, NH₂ | dideoxydehydro guanosine | HIV |
| NH₂, H | dideoxydehydro adenine | HIV |

(c) dioxolane purine derivatives of the formula 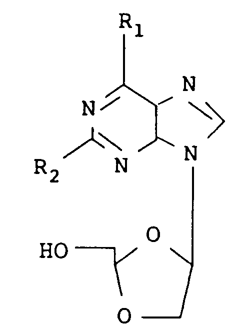 wherein the variations of R₁ and R₂ wherein R₁ is OH, or NH₂ and R₂ is H or NH₂, said compounds being targeted for HIV and HBV (i.e., hepatitis B), and
(d) oxetane type derivatives of the formula 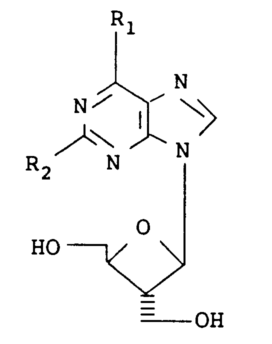 wherein R₁ is OH or NH₂ and R₂ is H or NH₂. The target viruses being HIV.

It is to be noted that the presently two best antiviral agents for hepatitis B (HBV) are dideoxyguanosine (ddGuo) and 2,6-diimino dideoxy purine riboside which is a "pro-drug" of dideoxyguanosine.

## Claims

1. Compounds of the formula 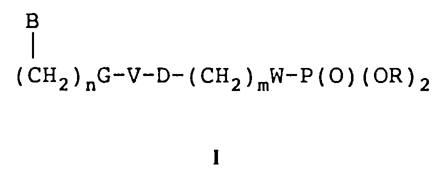 the isomeric and tautomeric forms thereof, and the pharmaceutically acceptable salts thereof, wherein B is a purine or pyrimidine moiety of the formulae 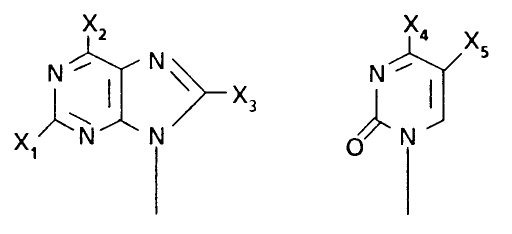 wherein
X₁ is H, NH₂, F, Cl, Br or I,
X₂ is H, OH, Cl, NH₂, SCH₃, SH, NHCH₃, or NHC₃₋₆ cycloalkyl,
X₃ is H, OH, NH₂, NHNH₂, CH₃, Cl, Br, or NHCH₃,
X₄ is NH₂, OH, OC₁₋₃ alkyl,
X₅ is H, CH₃, C₂H₅, I, F, Cl, Br, NHR, SR, C≡CH, CH=CH₂, CH=CHBr, CH₂CH₂Cl, or CH₂CH₂F,
R is H or C₁₋₈ alkyl,
m and n are zero, 1, 2 3 or 4,
V is 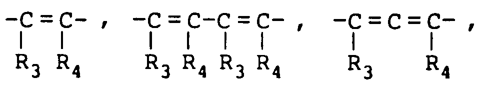 -C≡C- or 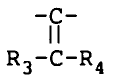
R₃ is H, F, Cl, CH₃, CH₂OH, CH₂F or OH,
R₄ is H, F, Cl, CH₃, CH₂OH, CH₂F or OH,
G is 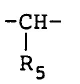 or is deleted,
D is 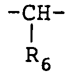 or is deleted with the proviso that at least one of G and D is deleted,
R₅ is F, Cl, CH₃, CH₂OH, OH or CH₂F with the proviso that when n is zero R₅ is other than OH, F or Cl, and
R₆ is F, Cl, CH₃, CH₂OH, OH or CH₂F,
W is OCH₂, CF₂, CCl₂, CHF, CHCl or is deleted.
